# EUROPEAN PATENT APPLICATION

(11) **EP 1 400 233 A1**
(43) Date of publication of application: **24.03.2004**
(21) Application number: 02738649.9
(22) Date of filing: 10.06.2002
(51) Int. Cl.: A61K 7/00, A61K 7/48

(54) **COSMETIC**

(30) Priority: 12.06.2001 JP 2001177184; 20.06.2001 JP 2001186516
(71) Applicant: Trial Corporation, Hadano-shi, Kanagawa 257-0015 (JP)
(72) Inventor: UEDA, Kazue, UNITIKA LTD., R&D CENTER, Uji-shi, Kyoto 611-0021 (JP); YAMADA, Kazunobu, UNITIKA LTD., R&D Center, Uji-shi, Kyoto 611-0021 (JP); YOSHIMURA, Kazuko, UNITIKA LTD., R&D Center, Uji-shi, Kyoto 611-0021 (JP)
(74) Representative: Kupecz, Arpad
(86) International application number: PCT/JP2002/005761
(87) International publication number: WO 2002/100357

(57) **Abstract**

A cosmetic product with excellent tactility and slip quality during skin application is provided. This cosmetic product includes microparticles and other cosmetic raw materials. The microparticles are formed from a biodegradable polyester resin containing 50 mol% or more of lactic acid units in relation to the resin component, with the content of D-lactic acid among the lactic acid units being 0.1 to 25 mol%. 70% or more in entire number of the microparticles satisfies the ratio of the large diameter to the small diameter as being equal to or less than 1.5. The density of the microparticles is 1.0 to 10.0 g/cm³, the average particle diameter is 0.01 to 1000 µm, and the 10% deformation strength during compression is 5 MPa or more.

## Description

### TECHNICAL FIELD

The present invention relates to a cosmetic product used for skin, hair, and the like, and particularly relates to a cosmetic product obtained using microparticles consisting of a biodegradable polyester resin.

### BACKGROUND ART

It is common practice to blend crosslinked silicone particles, nylon particles, vinyl or acryl radical polymer particles, or other such polymer microparticles with a cosmetic product as a cosmetic raw material in order to improve the tactility (moist or smooth feel) and slip quality during application of the cosmetic product on the skin, or to improve the slip quality and tactility during use of the cosmetic product for hair. Known examples of this type of cosmetic product include a cosmetic product containing crosslinked silicone particles having a three-dimensional mesh structure (see JP-A-1-165509 and JP-A-1-190757), a cosmetic product containing a silicone oil emulsion consisting of silicone crosslinked substances and the like (see JP-A-3-79669), a cosmetic product containing substantially spherical polyamide particles with a specific size (see JP-A-7-300410), a cosmetic product containing specific polymethyl methacrylate, polystyrene, or other such polymer microparticles (JP-A-10-502389), or the like.

However, these polymer microparticles are composed primarily from petroleum raw materials, and although their safety for skin use has been confirmed, they do not conform to the needs of consumers wanting to use safer products. These polymer microparticles also do not easily decompose in the natural environment, and when discarded into the natural environment after being used as a cosmetic product, the microparticles remain for a long time as waste and sediment, and have a large environmental impact, such as a considerable burden on furnaces when incinerated.

Therefore, biodegradable resins characterized by a reduced environmental impact have come to the fore. Despite restrictions in terms of physical properties, biodegradable resins have by now been processed and put to practical use as sheets, films, fibers, spunbonds, and the like. So far, the biodegradable resin foam disclosed in JP-A-5-170965 and elsewhere, the biodegradable resin water dispersions and emulsions disclosed in JP-A-9-78494, and the like have been studied as ways to processes biodegradable resins into particle configurations, and have been proposed as substitute products for resins manufactured from petroleum raw materials. Particularly, these have long been researched and put to practical use as capsules in so-called drug delivery applications, in which drugs are released in a sustained manner in living organisms and the like, as described in JP-B-1-5005 and elsewhere.

Several water dispersions of biodegradable resins have been proposed so far. A starch derivative is disclosed in JP-A-9-77910 and elsewhere; a polyhydroxy alkanoate in WO9704036; polycaprolactone in "Colloid Polymer Journal," vol. 273, p. 501 (1995), and an aliphatic polyester composed of an aliphatic dicarboxylic acid and glycol in JP-A-11-92712.

However, since all these biodegradable resins have low melting points or decomposition temperatures, the temperature range is limited during their use as a cosmetic product, and problems easily occur during storage or the like at high temperatures during the summer or the like. When microparticles are produced from an emulsified state by drying, only those with inferior mechanical strength can be obtained even if they can be made into spheres. Therefore, since these microparticles easily deform from spheres when added to a cosmetic product, and their rolling properties are readily reduced, they are unsuitable for use as materials with improved tactility or materials with improved slip quality during application to the skin or the like.

Of these biodegradable resins, polylactic acid has a relatively high melting point or softening point, at about 100 to 170°C, and possesses excellent water resistance, and is therefore suitable as a microparticulate raw material for cosmetics.

Active research has been conducted in the past on microencapsulation with the objective of sustained release of drugs in relation to polylactic acid water dispersions. For example, JP-A-63-122620, JP-A-5-58882, JP-A-6-72863, and JP-A-9-110678 disclose microencapsulation technology using a lactic acid polymer. These all utilize a method wherein an oil-in-water (O/W) type emulsion is formed by dissolving a lactic acid polymer in an organic solvent, dissolving or dispersing drugs therein, dripping or mixing the resulting solution or dispersion in a surfactant-containing aqueous solution, and stirring the product to induce phase inversion and emulsification. However, there arise such problems that since the organic solvent is used in large amounts, it is difficult to completely remove the solvent, and since the steps involved in distilling off and removing the solvent cannot be performed in a completely sealed system, the effects on the surrounding environment cannot be ignored. Also, this method makes it difficult to obtain relatively large particles exceeding 1 µm that can be used in a cosmetic product.

A method for producing microparticles of a polylactic acid resin in the form of an emulsion (aqueous solution) is also proposed (Japanese Patent Application No. 2000-370106), but in this case as well, when microparticles are produced from an emulsified state by drying, only those with inferior mechanical strength can be obtained even if they can be made into spheres. Therefore, they are unsuitable for being added to cosmetic products and used in applications involving materials with improved tactility or materials with improved slip quality during application. The improvement of mechanical strength is small even with methods in which the resin is fashioned into microparticles after being dissolved in an organic solvent.

Foamed particles can also be obtained by a method in which a biodegradable resin is pulverized, impregnated with an organic solvent or the like, and expanded. However, only foamed particles with a low density can be obtained, and these have problems with strength and specific gravity as a material for cosmetic products. Problems are also encountered in this case when an organic solvent is used.

JP-A-2000-220994 discloses biodegradable balls characterized in that a biodegradable material consisting of a thermoplastic resin with polylactic acid as the main ingredient is processed into spheres. However, particles with a large outside diameter of about 30 mm can be obtained with this technology, but it is impossible to produce particles with a diameter of 1 mm or less suitable as a material for cosmetic products.

Furthermore, even with all of the methods described above, it is extremely difficult to enclose inorganic, organic, or inorganic/organic composite substances in a resin in order to improve the characteristics of cosmetic products, and biodegradable polyester resin composite microparticles containing these cannot be obtained.

### DISCLOSURE OF THE INVENTION

The present invention was designed in view of the circumstances described above, and an object thereof is to provide a cosmetic product with excellent tactility and slip quality during application.

The cosmetic product of the present invention includes microparticles and other cosmetic raw materials. The microparticles are formed from a biodegradable polyester resin containing 50 mol% or more of lactic acid units in relation to the resin component, with the content of D-lactic acid among the lactic acid units being 0.1 to 25 mol%. 70% or more in entire number of the microparticles satisfies the ratio between the large diameter and the small diameter as being equal to or less than 1.5. The density of the microparticles is 1.0 to 10.0 g/cm³, the average particle diameter is 0.01 to 1000 µm, and the 10% deformation strength of the microparticles during compression is 5 MPa or more.

Such a cosmetic product has extremely high microparticle strength and is highly effective in improving tactility or improving slip quality during application to the skin or the like. Moreover, these microparticles can be easily and inexpensively manufactured without using an organic solvent and without using special equipment or complicated operations.

The cosmetic product of the present invention preferably contains 0.1 to 1000 ppm of lactic acid.

Lactic acid released by polylactic acid is thus present in the cosmetic product, making it easier to keep the skin mildly acidic and to obtain a cosmetic product that is mild on the skin.

In the cosmetic product of the present invention, the microparticles can contain 1 to 95% by volume of at least one type of component selected from inorganic substances and/or organic substances in addition to the biodegradable polyester resin, assuming that the microparticles in total constitute 100% by volume.

According to the present invention, the characteristics of the cosmetic product can be improved by adding at least one type of component selected from inorganic substances and/or organic substances in this manner.

In the method for manufacturing the cosmetic product of the present invention, a biodegradable polymer resin containing 90 mol% or more of lactic acid units is used, with the content of the D-form among the lactic acid units being 0.1 to 25 mol%. The biodegradable polyester resin is dried to a moisture content of 1000 ppm or less, melt kneading methods are applied to the dried resin to form microparticles, and the microparticles are added to the other cosmetic raw materials.

Thus, the biodegradable polyester resin with lactic acid as a primary component is dried to a moisture content of 1000 ppm or less, and melt kneading methods are applied to the dried resin to form microparticles, so the resulting microparticles have a small content of lactic acid monomers, and the lactic acid content of the cosmetic product can therefore be kept in a suitable range of 0.1 to 1000 ppm as described above.

### DETAILED DESCRIPTION OF THE INVENTION

The present invention will be described in detail below.

The biodegradable polyester resin that constitutes the microparticles used in the cosmetic product of the present invention is a lactic acid polymer comprising lactic acid units as a primary component, and by itself is essentially a hydrophobic polymer that does not disperse or dissolve in water. The constituent components of the biodegradable polyester resin will be described below.

Examples of the biodegradable polyester resin used in the present invention include homopolymers of lactic acid and copolymers of lactic acid with other hydroxycarboxylic acids, such as glycolic acid, 3-hydroxybutyric acid, 4-hydroxybutyric acid, 4-hydroxynaleric acid, 5-hydroxyvaleric acid, 6-hydroxycaproic acid, and the like. Other examples include copolymers of lactic acid with aliphatic carboxylic acids and glycolic acid. Additional examples include mixtures of polylactic acid with other polyhydroxycarboxylic acids, polycaprolactones, aliphatic polyesters, and the like.

The content of lactic acid units in the biodegradable polyester resin must be 50 mol% or more, and is preferably 70 mol% or more, and more preferably 90 mol% or more. When the content of lactic acid units is less than 50 mol%, the resin film obtained from the water dispersion has low heat resistance and is not practical for use as a cosmetic product.

The fact that lactic acid is released in a small amount by the polylactic acid has the effect of keeping the cosmetic product mildly acidic, and using this cosmetic product has the effect of keeping the skin mildly acidic. It is effective to have 0.1 to 1000 ppm of the released lactic acid in an aqueous solution at 30°C. This requires a biodegradable polyester resin comprising 50 mol% or more of lactic acid units as described above. When the lactic acid units in the biodegradable polyester resin are less than 50 mol%, the amount of lactic acid is less than 0.1 ppm and the acidity of the cosmetic product is insufficient, so the effect of keeping the skin mildly acidic is reduced when the cosmetic product is used. Conversely, when the amount of monomers remaining in the polylactic acid is too great, the amount of lactic acid in the cosmetic product is greater than 1000 ppm, a level with adverse effects on the skin. Therefore, the monomers present in the polylactic acid must be reduced as much as possible and the amount of lactic acid in the cosmetic product must be 1000 ppm or less, as described above.

The content of D-lactic acid in the lactic acid units must be 0.1 to 25 mol%, and is preferably 0.5 to 20 mol%, and more preferably 1 to 15 mol%. It is fairly difficult to form polylactic acid microparticles in which the D-form content is less than 0.1 mol%. Conversely, an amount of D-lactic acid greater than 25 mol% results in an increase of harmful effects, such as the glass transition temperature is lowered, the heat resistance of the resulting microparticles required for use as a cosmetic product is reduced, and costs increase.

In the present invention, the 10% deformation strength (method of measurement to be described later) during compression of the biodegradable polyester resin microparticles must be 5 MPa or more, and is preferably 10 MPa or more. When the 10% deformation strength is less than 5 MPa, the original spheres easily deform to become aspherical when blended with the cosmetic product, which reduces the effects of improved tactility and improved slip quality during application.

The biodegradable polyester resin used in the cosmetic product of the present invention preferably has a number-average molecular weight as measured by GPC (gel permeation chromatography, polystyrene conversion) in a range of 2,000 to 1,000,000. At less than 2,000, sufficient strength cannot be obtained, and the concentration of lactic acid in the cosmetic product therefore becomes too high due to the decomposition of polylactic acid and the production of lactic acid. It is difficult to procure a high polymer in excess of 1,000,000.

It is preferable to use composite microparticles in which inorganic, organic, or inorganic/organic composite substances are enclosed in a biodegradable polyester resin with polylactic acid as the main component in order to improve the characteristics of the cosmetic product.

The inorganic/organic compounds capable of forming such composite microparticles are not particularly limited as long as they can remain stable in the biodegradable polyester resin, and inorganic compounds, organic compounds, and inorganic/organic composite substances can be used.

Possible examples of the inorganic compound that can be used to improve the characteristics of the cosmetic product include metals, metal compounds, ceramics, and the like. Specific examples include aluminum, silicon, titanium, iron, nickel, copper, zinc, selenium, zirconium, molybdenum, ruthenium, palladium, silver, indium, tin, antimony, platinum, gold, bismuth, and the like. Other examples include oxides, nitrides, halides, carbonic acid compounds, hydroxide compounds, phosphoric acid compounds, and sulfur compounds of these elements and of elements including the first through third groups of the periodic table. More examples include composites, alloys, and natural/synthetic minerals of these compounds.

Possible examples of the organic compound include various synthetic resin compositions, oligomers, natural compounds, carbon compounds, and the like. Specifically, these are polyethylene, polypropylenes, and other such hydrocarbon resins, acrylic acid resins, vinyl acetate resins, diene compounds, polyethers, phenol resins, amino resins, aromatic hydrocarbon resins, polyester resins, polyamide resins, silicone resins, furan resins, polyurethane resins, epoxy resins, cellulose compounds, proteins, amino acids, carbides thereof, and the like. Of these, it is preferable to use a naturally occurring compound or a compound displaying biodegradability. One or more of these compounds may be used, and inorganic/organic compounds may also be mixed and used.

Furthermore, to classify the inorganic/organic compounds used when composite microparticles are produced in terms of the functions useful for a cosmetic product, it is possible to name substances that absorb or scatter ultraviolet rays, pigments, dyes, IR absorbers, absorbents for electromagnetic waves or radioactive rays, or the like. Generally these can be classed as coloring pigments, extender pigments, mixtures of coloring pigments, extender pigments coated with coloring pigments, or the like.

Possible examples of a coloring pigment include titanium oxide (titanium white, titanium black), zinc oxide, iron oxide (Indian red, yellow iron oxide, black iron oxide, ultrafine particulate iron oxide), alumina, aluminum hydroxide, zirconium oxide, titanium nitride, zirconium nitride, selenium oxide, silicon crabide, silicon nitride, boron carbide, boron nitride, strontium aluminate, zinc sulfide, and the like. Possible examples of the extender pigment include talc, kaolin, mica, sericite, loess, amber, titanium dioxide, zinc oxide, montmorillonite, mica, clay, bentonite, and the like. The extender pigment mixture can be a simple mixture or a coloring pigment coated with another coloring pigment, and specific examples include mica coated with titanium dioxide, and mica-titanium coated a least one of the following: Indian red, black iron oxide, ultramarine, navy blue, carbon black, titanium black, and the like. Examples of extender pigments coated with coloring pigments include mica coated with titanium dioxide and extender pigments coated with at least one of the following: Indian red, black iron oxide, ultramarine, navy blue, carbon black, titanium black, and the like.

The various compounds (hereinafter referred to as "compositing compounds") as described above for use in the composite microparticles may be liquids or gases, but solid substances are easiest to handle in terms of operability. When solid compositing compounds are used, their initial particle diameter is not particularly limited. However, if the initial particle diameter is large, it is preferable to use a compound whose particles are made into fine grains during manufacture of the composite microparticles. The content of the compositing compound must be 1 to 95% by volume of all the biodegradable polyester resin composite particles produced, and this compositing compound preferably has a structure in which the compound is covered with a biodegradable polyester to prevent direct contact with the skin. The improvement in functions due to compounding is insufficient when the content of the compositing compound accounts for less than 1% by volume of all the composite microparticles in the biodegradable polyester resin, and when it is greater than 95% by volume, it is difficult to cover with biodegradable polyester.

The biodegradable polyester resin microparticles (including composite microparticles) used in the cosmetic product of the present invention are manufactured by the following method.

A biodegradable polyester resin with polylactic acid as a primary component, or a biodegradable polyester resin composition consisting of a biodegradable polyester resin with polylactic acid as a primary component and at least one type of compositing compound selected from an inorganic and/or organic substance, is dried such that the moisture content is 1000 ppm or less. A moisture content greater than 1000 ppm is undesirable because in this case the biodegradable polyester causes a reduction in molecular weight and a large amount of lactic acid monomer remains in the microparticles produced.

Next, the biodegradable polyester resin or the biodegradable polyester resin composition (hereinafter the combination of both is sometimes referred to as "the biodegradable polyester resin composition" or simply "the composition" for the sake of simplicity) is heat melted and mixed with an incompatible dispersion medium at a temperature equal to or greater than the melting point of the biodegradable polyester resin composition, and this composition is dispersed into microparticles. Microparticles of the resulting biodegradable polyester resin composition are cooled to a temperature equal to or less than the melting point, and are solidified in the dispersion medium along with the dispersion medium as microscopic spheres with an average particle diameter of about 0.01 µm or greater and 1,000 µm or less. Thus, biodegradable polyester resin microparticles are manufactured.

The amount of dispersion medium used herein is preferably one to five times the volume of the biodegradable polyester resin composition.

The heating temperature during mixing should be higher than the melting point of the substance with the highest melting point of all the substances that constitute the biodegradable polyester resin composition, and it is preferable to use a temperature 10 to 200°C higher than the melting point, and more preferably a temperature 20 to 100°C higher. When the heating temperature is too low, the biodegradable polyester resin composition does not sufficiently melt and does not easily disperse into microparticles. Nor is it suitable to set the heating temperature too high, because it causes the biodegradable polyester resin composition to undergo thermal decomposition, a reduction in molecular weight, or the like, and is also not preferable from the perspective of lactic acid monomers remaining in the resin.

After the microparticles are formed by cooling and solidifying, a solvent that is a poor solvent (precipitant) for the biodegradable polyester resin composition and a good solvent for the dispersion medium (hereinafter referred to as "suspension-producing solvent") is added to and mixed with the mixed solid of the microparticulate biodegradable polyester resin composition and the dispersion medium. The dispersion medium is thereby dissolved to make a suspension of the biodegradable polyester resin composition, and the desired microparticles are separated and extracted from this suspension.

Possible examples of hydrophilic substances that can be used as such a dispersion medium include at least one of the following: polyalkylene oxides (polyethylene glycol and the like), homopolymers or copolymers of polyalkenecarboxylic acids, or salts thereof (polyacrylic acid, polymethacrylic acid, sodium polyacrylate, and the like), homopolymers or copolymers of polyalkeneamides (polyacrylamide, polymethacrylamide), and the like. Examples of the hydrophobic substances include propyl cellulose and polystyrene.

When the biodegradable polyester resin microparticles or composite microparticles used in the cosmetic product of the present invention are manufactured, the methods and devices for mixing the biodegradable polyester resin or resin composition with the dispersion medium and dispersing the resulting product in the dispersion medium as microparticles are not particularly limited. For example, the materials may be dispersed with the aid of rollers, a Banbury mixer, a kneader, a single-screw extruder, a twin-screw extruder, or the like. Among these, it is preferable to use a single-screw or twin-screw extruder because of considerations related to continuous productivity and increased microparticle strength during compression, and it is particularly preferable to use a twin-screw extruder capable of melting and kneading a resin or resin composition and a dispersion mecium under high shear conditions. The type and size of the twin-screw extruder are not particularly limited, but one with a ratio of cylinder length to bore diameter (L/D) of 20 or more can be preferably used.

This type of granulation method is considered to belong to wet stirring and granulation, and the particle size is believed to be determined by the balance between the stirring-induced shear force, which is the force that splits the microparticles, and the viscoelasticity and surface tension of the composition, which are the forces that preserve the microparticles.

When the biodegradable polyester resin microparticles or composite microparticles are manufactured using the preferred methods previously described, the biodegradable resin molecules are very finely dispersed in the dispersion medium by the high shear force applied in the process. Moreover, because of such high shear force, it is presumed that the microparticles obtained here have a particularly superior compression strength compared to microparticles obtained by other processes.

As described above, the resin composition/dispersion medium mixture is cooled to the melting point or less and solidified, and the suspension-producing solvent, which is a poor solvent for the composition and a good solvent for the dispersion medium, is then mixed with this mixture, although this mixture may also re used in unaltered form as the suspension of the microparticles in obtaining the cosmetic product of the present invention. Alternatively, it is also possible to adopt a process in which the mixture is cooled and solidified; pulverized with a crusher or the like, pelletized with a pelletizer, or molded into a sheet with an extruder, roll, or the like; mixed with the suspension-producing solvent; and advanced to the suspension-producing step.

Water or an aqueous organic solvent can be used as the suspension-producing solvent for the hydrophilic dispersion medium. For example, when the dispersion medium is a polyalkylene oxide or polyalkylene carboxylic acid, water can be used as the suspension-producing solvent, as described above.

A hydrophobic organic solvent can be used as the suspension-producing solvent for the hydrophobic dispersion medium. For example, when propyl cellulose is used as the dispersion medium, toluene, methylethyl ketone (MEK), or the like, which are good solvents thereof, can be used.

However, in view of effects on the environment, it is preferable to use a hydrophilic dispersion medium and water, as described above.

The desired resin microparticles can be separated from the suspension of the resin/dispersion medium mixture by centrifugal separation, filtration, or a combination of these methods. The separated microparticles are used after being dried as necessary.

Surface treatment may be further performed on the microparticles obtained as described above in order to improve the characteristics of the cosmetic product. A method of coating a metal oxide (oxide of iron, zinc, aluminum, zirconium, cerium, cobalt, or the like) using a wet pulverization device can be employed as the method of surface treatment (JP-A-8-59433).

The microparticles or composite microparticles used in the cosmetic product of the present invention must be formed into easily rolling spheres in order to contribute to the improvement of tactility of the cosmetic product and to the improvement of slip quality during application to the skin or the like. The conditions for these spheres are such that those whose ratio of the large diameter to the short diameter is 1.5 or less must make up 70% or more of all the microparticles. When this ratio is greater than 1.5, the spheres become elongated and do not roll easily, their strength decreases, and they tend to become even more aspherical. Therefore, when spheres with a ratio of 1.5 or less make up less than 70% of all the spheres, all the microparticles have insufficient rolling properties and strength.

The density of the microparticles must be 1.0 to 10 g/cm³. A density of less than 1.0 g/cm³ means that there are voids in the microparticles, and the strength declines. A density greater than 10 g/cm³ increases the difference in density with the other materials constituting the raw materials of the cosmetic product, creating problems during kneading and the like.

The average diameter of the microparticles must be 0.01 to 1,000 µm. Among these, particles with an average diameter of 1 to 100 µm are preferably used in numerous applications as cosmetic products. Particles with an average diameter less than 0.01 µm are difficult to form and have a poor yield rate. They are also subject to static electricity and moisture absorption and are difficult to handle as a powder. On the other hand, an average particle diameter exceeding 1,000 µm results in a poor yield rate and increased costs.

The cosmetic product of the present invention obtained using the microparticles will be described in detail. The cosmetic product of the present invention is characterized by consisting of biodegradable polyester resin microparticles or biodegradable polyester resin composite microparticles and another cosmetic raw material. Examples of the types of cosmetic products of the present invention include soap, body shampoo, facial cleansing cream, and other such cleansing cosmetic products; skin toners, creams, emulsions, packs, and other such basic skin care products; powders, foundations, and other such base makeup products; lipstick, blusher, eye shadow, eye liner, mascara, and other such facial cosmetic products; makeup products for manicures and the like; shampoos, hair rinses, hair conditioners, hair treatments, setting lotions, blow styling lotions, hairsprays, foam styling agents, gel styling agents, hair liquid, hair tonic, hair cream, hair growth tonic, hair growth stimulants, hair dyes, hair dressing, and other such hair care cosmetic products; perfumes, eau de colognes, and other such aromatic cosmetic products; toothpastes; bath preparations; and depilatories, aftershave lotions, antiperspirants, deodorants, sun blocks, and other such special cosmetic products. Basic skin care products, makeup products, and other such skin cosmetic products or hair care cosmetic products are particularly preferred. Examples of the physical states of this cosmetic product include aqueous liquid, oil-based liquid, emulsion, cream, foam, semisolid, solid, and powder. It is also possible to use this cosmetic product by spraying.

This cosmetic product consists of the biodegradable polyester resin microparticles or the biodegradable polyester resin composite microparticles and other cosmetic raw materials, and examples of the other cosmetic raw materials include avocado oil, almond oil, olive oil, cacao butter, sesame oil, wheat germ oil, safflower oil, shea butter, turtle oil, camellia oil, persic oil, castor oil, grape oil, macadamia nut oil, mink oil, egg yolk oil, Japan tallow, coconut oil, rose hip oil, hydrogenated oil, and other such oils and fats; orange roughy oil, carnauba wax, candelilla wax, whale wax, jojoba oil, montan wax, beeswax, lanolin, and other such waxes; liquid paraffin, vaseline, paraffin, ceresin, microcrystalline wax, squalane, and other such hydrocarbons; lauric acid, myristic acid, palmitic acid, stearic acid, oleic acid, behenic acid, undecylenic acid, oxystearic acid, linoleic acid, lanolic acid, synthetic fatty acids, and other such higher fatty acids; ethyl alcohol, isopropyl alcohol, lauryl alcohol, cetyl alcohol, cetostearyl alcohol, stearyl alcohol, oleyl alcohol, behenyl alcohol, lanolin alcohol, hydrogenated lanolin alcohol, hexyl decanol, octyl dodecanol, isostearyl alcohol, and other such alcohols; cholesterol, dihydrocholesterol, phytosterol, and other such sterols; ethyl linoleate, isopropyl myristate, lanolin fatty acid isopropyl, hexyl laurate, myristyl myristate, cetyl myristate, octyldodecyl myristate, decyl oleate, octyldodecyl oleate, hexyldecyl dimethyl octanoate, cetyl isooctanoate, cetyl palmitate, glycerin trimyristate, glycerin tri(capryl-caprate), propylene glycol dioleate, glycerin triisostearate, glycerin triisooctanoate, cetyl lactate, myristyl lactate, diisostearyl malate, and other such fatty acid esters; glycerin, propylene glycol, 1,3-butylene glycol, polyethylene glycol, d,1-sodium pyrrolidone carboxylate, sodium lactate, sorbitol, sodium hyaluronate, and other such humectants; higher fatty acid soaps, higher alcohol sulfuric acid ester salts, N-acyl glutamate, phosphoric acid ester salts, and other such anionic surfactants; cationic surfactants; betaine surfactants, amino acid surfactants, imidazoline surfactants, lecithin, and other such amphoteric surfactants; polyhydric alcohol ester surfactants, ethylene oxide condensed surfactants, and other such nonionic surfactants, as well as other surfactants; iron oxide and other such colored pigments; zinc oxide, titanium oxide, zirconium oxide, and other such white pigments; mica, talc, sericite, and other such extender pigments; dimethyl polysiloxane, methylphenyl polysiloxane, octamethyl tetracyclosiloxane, decamethyl cyclopentasiloxane, polyether-modified silicone oil, amino-modified silicone oil, and other such silicone oils; purified water; carrageenan, alginic acid, gum arabic, gum tragacanth, pectin, starch, xanthan gum, polyvinyl alcohol, polyvinyl pyrrolidone, sodium pclyacrylate, polyethylene glycol, and other such thickening agents; and silicone/acryl copolymers, silicone resins, acryl polymers, and other such film-forming agents; and also ultraviolet absorbers, antibacterial agents, anti-inflammatory agents, antiperspirants, preservatives, perfumes, antioxidants, pH regulators, and propellants.

The method for manufacturing the cosmetic product of the present invention will be described. The method for manufacturing the cosmetic product of the present invention is characterized in that the biodegradable polyester resin microparticles or biodegradable polyester resin composite microparticles are blended with other cosmetic raw materials. Blending can be performed, for example, by batch-wise or continuous mixing means. Examples of a specific device for this purpose include a homomixer, a paddle mixer, a Henschel mixer, a homodisperser, a colloid mill, a propeller mixer, a homogenizer, an inline continuous emulsion machine, an ultrasonic emulsion machine, and a vacuum-kneading machine. The biodegradable polyester resin microparticles or biodegradable polyester resin composite microparticles may be added to the other cosmetic raw material at any time.

In the method for manufacturing the cosmetic product of the present invention, the added amount of biodegradable polyester resin microparticles or biodegradable polyester resin composite microparticles is preferably 0.1 to 99% by mass in relation to the cosmetic product in terms of components other than moisture (in terms of solid fraction), and is particularly preferably in a range of 0.5 to 90% by mass. This is because the cosmetic product tends to become ineffective as a cosmetic product when the added amount of the biodegradable polyester resin microparticles or biodegradable polyester resin composite microparticles exceeds the upper limit of the range described above, and if the added amount is less than the lower limit of the range described above, the addition effect tends to be small and it is more difficult to improve the organoleptic application attributes or other properties of the cosmetic product by blending the microparticles.

### Examples

The present invention will be described in detail below by examples.

The items listed below were analyzed according to each of the methods described therein.

### (1) Molecular weight of biodegradable polyester resin

The molecular weight was determined by GPC analysis (refractive index spectrometer made by Shimadzu Corp., solvent: tetrahydrofuran, in terms of polystyrene).

### (2) Measuring content of lactic acid units in relation to the resin component of microparticles

Determined by dissolving microparticles in D-chloroform and conducting NMR measurement (Lambda 300WB made by JEOL) at room temperature.

### (3) Measuring content of the D-form in the lactic acid units of microparticles

The microparticles were dissolved in chloroform, specific optical rotation was calculated for the Na-D line (589 nm) by using a polarimeter (SEPA-200 made by Horiba, Ltd.) at 25°C, and the content of the D-form (mol%) was determined.

### (4) Measuring major diameter and minor diameter of microparticles

Microparticles with a diameter of 5 µm or greater were measured at a magnification of 500 using an optical microscope (PM-10AK made by Olympus Corp.). Microparticles with a diameter less than 5 µm were measured at a magnification of 2000 to 5000 using a scanning electron microscope (S-4000 made by Hitachi, Ltd.).

Then, the number of microparticles with a ratio of major diameter to the minor diameter of 1.5 or less present in a specified number (denoted as "object of measurement" in Table 1 below) of microparticles in the field of vision of the microscope were counted to determine their percentage in the total number of microparticles.

### (5) Density of microparticles

A saltwater-based density gradient tube was manufactured, and corrections were made and measurements performed using a standard density sample.

### (6) 10% deformation strength of microparticles

After measuring the diameter of each microparticle by microscopic observation, the strength was measured at a test load of 49 mN and a load rate of 0.89 mN/s using a micro-compression testing machine (MCTM-500 made by Shimadzu Corp.). The compression load at the time the particle diameters changed by 10% was taken to be the 10% deformation strength. Each sample was measured ten times and the average value was used.

### (7) Average particle diameter of microparticles

Measured using a particle size analyzer (LA920 made by Horiba Ltd.) and evaluated with the volume-average particle diameter.

### (8) Determining the lactic acid content of cosmetic product

Determined by HPLC (column: Amix HPX-87P, 300 mm (in diameter) × 7.8 mm, mobile tank: 0.005 N sulfuric acid, detection: RI, temperature: 60°C, flow rate: 0.6 ml/min, injected sample: 100 µl. The standard sample was a 1/500 diluted aqueous solution of lactic acid (reagent grade, Ishizu Seiyaku Co., Ltd.)). The content of lactic acid in cosmetic products was measured after they were allowed to stand for one month in an atmosphere of 30°C.

### (9) Evaluation of cosmetic product

An organoleptic test was conducted with twenty panelists. Tactility and slip quality were selected as the items to be evaluated in this test, and each item was evaluated in five steps by the following standards.

### 1: poor, 2: somewhat poor, 3: normal, 4: fair, 5: good

The pH of the skin during application of the cosmetic product was also measured using a skin pH meter-PH900 (made by Courage + Khazaka electronic GmbH).

Biodegradable polyester resin microparticles (A) through (H) were obtained according to the procedure described below.

### Microparticles (A)

40 parts by mass of polylactic acid (number-average molecular weight: 60,000; content of the D-form: 1.7 mol%) as a biodegradable polyester resin dried until the moisture content reached 200 ppm, and 60 parts by mass of polyacrylic acid (weight-average molecular weight: 1,000,000) as a dispersion medium were dry blended and fed to the feed port of a one-way twin-screw extruder (PCM-30 made by Ikegai Tekko). The cylinder temperature of the extruder was set to 180°C, the materials were melted and kneaded, and the resin composition was extruded from the nozzle, cooled, and solidified. The polyacrylic acid was then dissolved using an amount of water ten times the mass of the polyacrylic acid, and a suspension of polylactic acid spherical particles about 10 µm in diameter was obtained. This suspension was centrifuged and dried to obtain biodegradable polyester resin microparticles (A).

### Microparticles (B)

4 parts by mass of a fine powder of zinc oxide were added as a compositing compound for compounding the composite microparticles with 40 parts by mass of polylactic acid (number-average molecular weight: 60,000; content of the D-form: 1.7 mol%) as a biodegradable polyester resin dried until the moisture content reached 200 ppm, the resulting product was thoroughly mixed with 60 parts by mass of polyethylene glycol (P20000 made by Sanyo Chemical) as a dispersion medium, and the materials were fed to the feed port of a one-way twin-screw extruder (PCM-30 made by Ikegai Tekko). The cylinder temperature of the extruder was set to 200°C, the materials were melted and kneaded, and the resin composition was extruded from the nozzle, cooled, and solidified. The polyethylene glycol was then dissolved using an amount of water ten times the mass of the polyethylene glycol, and a suspension of polylactic acid spherical particles containing zinc oxide about 10 µm in diameter was obtained. This suspension was centrifuged and dried to obtain biodegradable polyester resin composite microparticles (B).

### Microparticles (C)

The polylactic acid used had a number-average molecular weight of 100,000 and a D-form content of 9 mol%, and it was dried until the moisture content reached 200 ppm. A pressure-kneading device (PK5 made by Moriyama Manufacturing Co.) was used instead of the one-way twin-screw extruder. Otherwise the procedure was conducted identical to that of microparticles (A) to obtain biodegradable polyester resin microparticles (C).

### Microparticles (D)

The polylactic acid used had a number-average molecular weight of 100,000 and a D-form content of 19 mol%, and it was dried until the moisture content reached 200 ppm. The cylinder temperature of the twin-screw extruder was set to 170°C. Otherwise the procedure was conducted identical to that of microparticles (A) to obtain biodegradable polyester resin microparticles (D).

### Microparticles (E)

A mixture of 1.8 kg of polylactic acid (number-average molecular weight: 100,000; D-form content 19 mol%) as a biodegradable polyester resin dried until the moisture content reached 200 ppm, and 0.2 kg of polycaprolactone (number-average molecular weight: 80,000) as a compositing compound was used. The cylinder temperature of the twin-screw extruder was set to 160°C. Otherwise the procedure was conducted identical to that of microparticles (A) to obtain biodegradable polyester resin microparticles (E).

### Microparticles (F)

A fine powder of titanium white was used instead of zinc oxide powder as the compositing compound. Otherwise the procedure was conducted identical to that of microparticles (B) to obtain biodegradable polyester resin composite microparticles (F).

### Microparticles (G)

Starch powder (potato starch) was used instead of zinc oxide powder as the compositing compound. Otherwise the procedure was conducted identical to that of microparticles (B) to obtain biodegradable polyester resin composite microparticles (G).

### Microparticles (H)

A fine powder of ferrite was used instead of zinc oxide powder as the compositing compound. Otherwise the procedure was conducted identical to that of microparticles (B) to obtain biodegradable polyester resin composite microparticles (H).

### Example 1

20 parts by mass of biodegradable polyester resin microparticles (A), 5 parts by mass of p-octyl methoxycinnamate, 1 part by mass of α-monoisostearyl glyceryl ether polyoxyethylene sorbitan ester monooleic acid ester as a fatty acid ester, 2 parts by mass of beeswax as a wax, 2 parts by mass of lanolin as a wax, 20 parts by mass of squalane as a hydrocarbon, 30 parts by mass of liquid paraffin as a hydrocarbon, 19 parts by mass of purified water, an adequate amount of a preservative, and an adequate amount of perfume were placed in a homodisperser and mixed for 3 minutes at 2500 rpm to prepare an emulsified cosmetic product.

### Example 2

30 parts by mass of biodegradable polyester composite resin microparticles (B), 5 parts by mass of p-octyl methoxycinnamate, 1 part by mass of α-monoisostearyl glyceryl ether polyoxyethylene sorbitan monooleic acid ester, 2 parts by mass of beeswax, 2 parts by mass of lanolin, 20 parts by mass of squalane, 20 parts by mass of liquid paraffin, 19 parts by mass of purified water, an adequate amount of a preservative, and an adequate amount of perfume were placed in a homodisperser and mixed for 3 minutes at 2500 rpm to prepare an emulsified cosmetic product.

### Example 3

30 parts by mass of biodegradable polyester resin microparticles (C), 5 parts by mass of p-octyl methoxycinnamate, 1 part by mass of α-monoisostearyl glyceryl ether polyoxyethylene sorbitan monooleic acid ester, 2 parts by mass of beeswax, 2 parts by mass of lanolin, 20 parts by mass of squalane, 20 parts by mass of guar gum as a thickening agent, 19 parts by mass of purified water, an adequate amount of a preservative, and an adequate amount of perfume were placed in a homodisperser and mixed for 3 minutes at 2500 rpm to prepare an emulsified cosmetic product.

### Example 4

30 parts by mass of biodegradable polyester resin microparticles (D) were used. Otherwise the procedure was conducted identical to that of Example 3 to prepare an emulsified cosmetic product.

### Example 5

30 parts by mass of biodegradable polyester resin microparticles (E) were used. Otherwise the procedure was conducted identical to that of Example 3 to prepare an emulsified cosmetic product.

### Example 6

30 parts by mass of biodegradable polyester resin composite microparticles (F) were used. Otherwise the procedure was conducted identical to that of Example 3 to prepare an emulsified cosmetic product.

### Example 7

30 parts by mass of biodegradable polyester resin composite microparticles (G) were used. Otherwise the procedure was conducted identical to that of Example 3 to prepare an emulsified cosmetic product.

### Example 8

30 parts by mass of biodegradable polyester resin composite microparticles (H) were used. Otherwise the procedure was conducted identical to that of Example 3 to prepare an emulsified cosmetic product.

### Comparative Example 1

1 kg of polylactic acid (number-average molecular weight: 60,000; D-form content: 1.7 mol%) dried until the moisture content reached 200 ppm was dissolved in 10 kg of methylene chloride and added to water under stirring to obtain a suspension of polylactic acid spherical particles. The suspension was subjected to centrifugal separation and dried to obtain biodegradable polyester resin microparticles (a).

30 parts by mass of these microparticles (a) were used instead of microparticles (C). Otherwise the procedure was conducted identical to that of Example 3 to prepare an emulsified cosmetic product.

### Comparative Example 2

Polylactic acid (number-average molecular weight: 100,000; D-form content: 9 mol%) dried until the moisture content reached 200 ppm was used as the polylactic acid. Otherwise the procedure was conducted identical to that of Comparative Example 1 to obtain biodegradable polyester resin microparticles (b).

30 parts by mass of these microparticles (b) were used instead of microparticles (a). Otherwise the procedure was conducted identical to that of Example 3 to prepare an emulsified cosmetic product.

### Comparative Example 3

250 g of partially depolymerized polylactic acid (number-average molecular weight: 20,000; D-form content: 9 mol%) dried until the moisture content reached 200 ppm was used as the polylactic acid, and the acid was swelled with 100 g of isopropyl alcohol. The swollen polylactic acid, 100 g of an aqueous solution containing 2% by mass of PVA (UF170G made by Unitika) as a surfactant, 550 g of distilled water, and an amount of triethylamine equivalent to 1.2 times the amount of all the carboxyl groups contained in the polylactic acid were introduced and vigorously stirred at 6,000 rpm. The temperature in the system was raised to 60°C and mixing was continued to obtain an emulsion. The emulsion was subjected to centrifugal separation and dried to obtain biodegradable polyester resin microparticles (c).

30 parts by mass of these microparticles (c) were used instead of microparticles (C). Otherwise the procedure was conducted identical to that of Example 3 to prepare an emulsified cosmetic product.

### Comparative Example 4

Polylactic acid (number-average molecular weight: 60,000; D-form content: 1.7 mol%) with a moisture content of 1200 ppm was used. Otherwise the procedure was conducted identical to that of polyester resin microparticles (A) to obtain biodegradable polyester resin microparticles (d).

These microparticles (d) were used instead of microparticles (A). Otherwise the procedure was conducted identical to that of Example 1 to prepare an emulsified cosmetic product.

Various measurements were taken of the biodegradable polyester resin microparticles and cosmetic products in Examples 1-8 and Comparative Examples 1-4. The results are shown in Table 1. The results of evaluating the cosmetic products and the results of measuring the pH state of the skin are shown in Table 2 for Examples 1-6 and in Table 3 for Examples 7-8 and Comparative Examples 1-4.

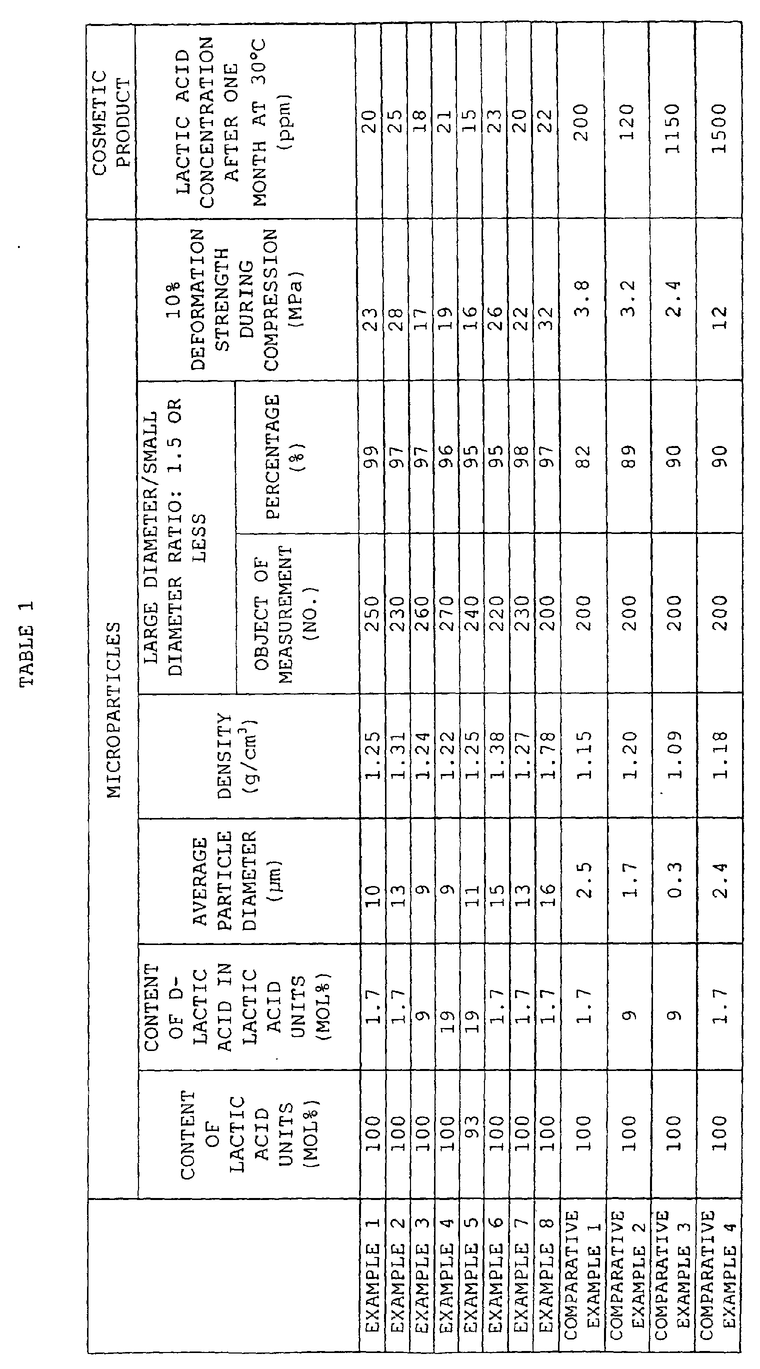

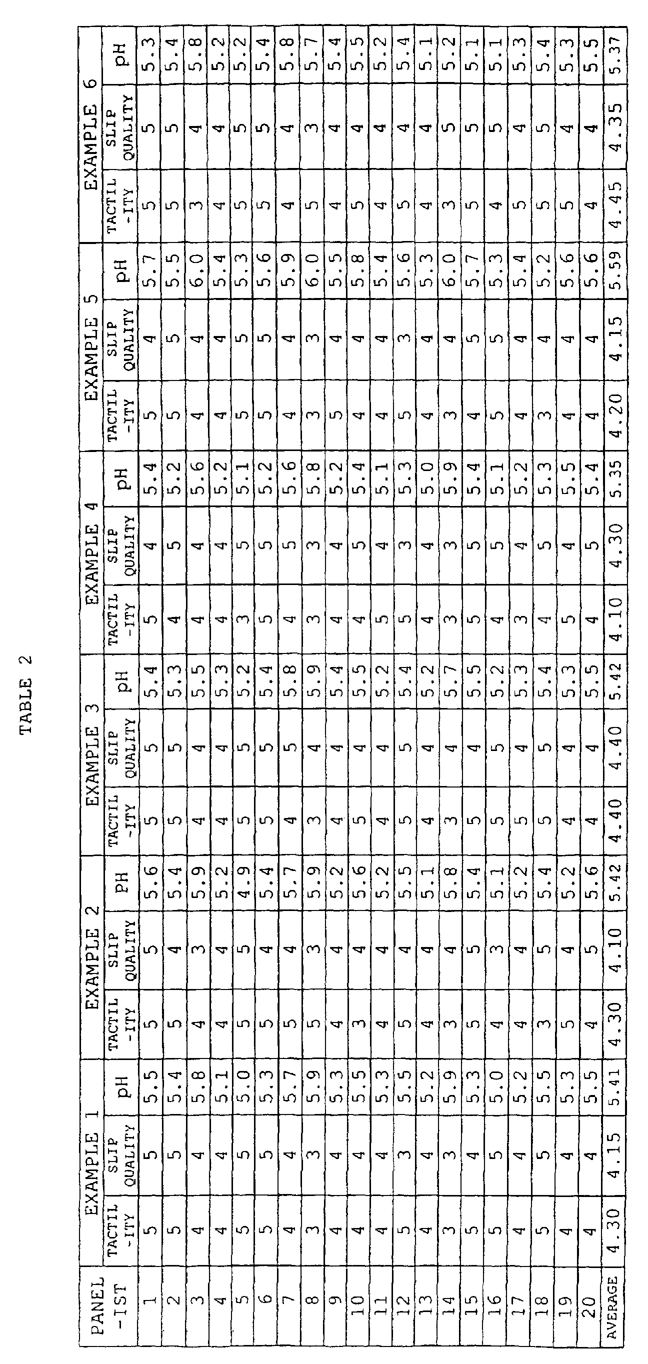

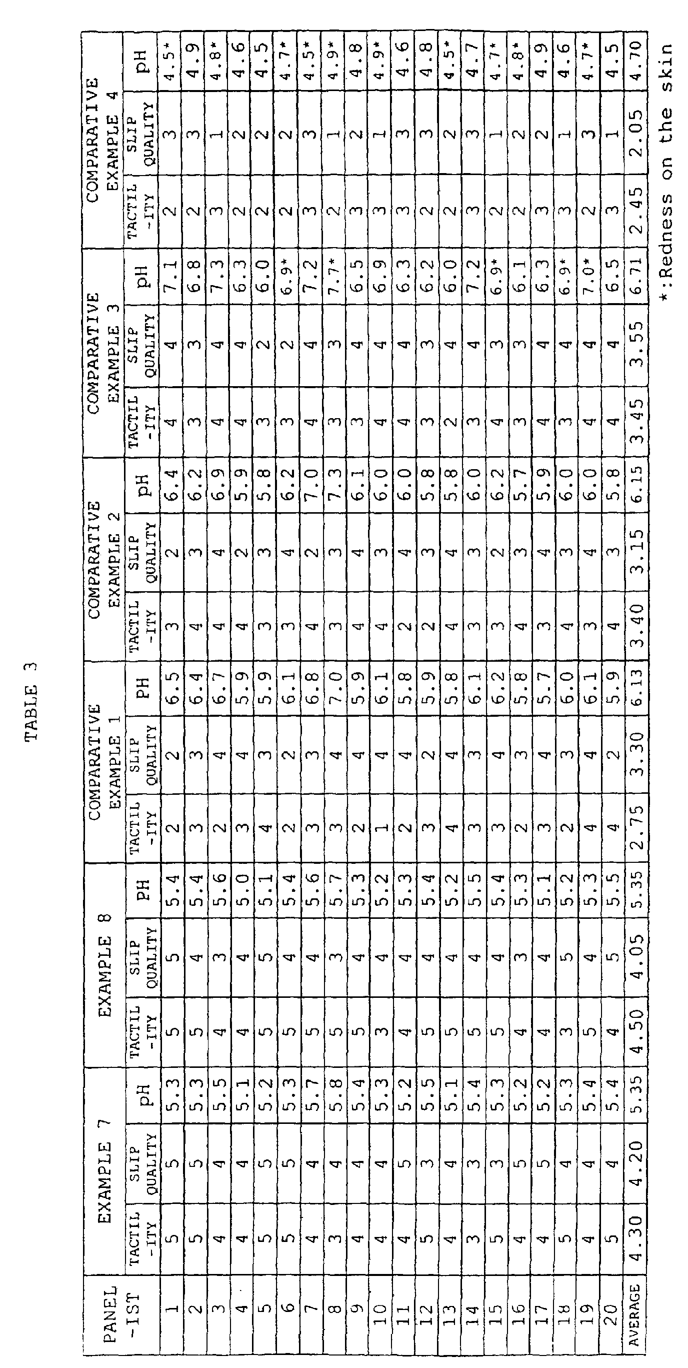

As is clear from the results in Tables 1 through 3, the cosmetic products in Examples 1 through 8 of the present invention have biodegradable polyester resin microparticles or biodegradable polyester resin composite microparticles with specific characteristics. Therefore, the resulting cosmetic products have excellent tactility and slip quality during skin application.

Conversely, the cosmetic products in Comparative Examples 1 through 3 do not have microparticles manufactured by melting and kneading raw material under high shear conditions as with the cosmetic products in Examples 1 through 8, so the microparticles have a low deformation strength during compression, and change shape when used as a cosmetic product, resulting in inferior tactility on the skin and slip quality during application.

The cosmetic product of Comparative Example 4 used polylactic acid with a moisture content of 1200 ppm, so a large amount of lactic acid monomer remained in the resulting microparticles, causing the lactic acid concentration of the resulting cosmetic product to reach 1500 ppm, and the cosmetic product to become unsuitable for use.

## Claims

1. A cosmetic product comprising microparticles and other cosmetic raw materials, wherein
the microparticles are formed from a biodegradable polyester resin containing 50 mol% or more of lactic acid units in relation to the resin component, with a content of D-lactic acid among the lactic acid units being 0.1 to 25 mol%;
70% or more in entire number of the microparticles satisfies a ratio between a large diameter thereof and a small diameter thereof as being (large diameter)/(small diameter) ≤ 1.5;
density of the microparticles is 1.0 to 10.0 g/cm³;
average particle diameter of the microparticles is 0.01 to 1000 µm; and
10% deformation strength of the microparticles during compression is 5 MPa or more.

2. The cosmetic product as set forth in claim 1, wherein 0.1 to 1000 ppm of lactic acid is contained.

3. The cosmetic product as set forth in claim 1, wherein assuming that the microparticles in total constitute 100% by volume, the microparticles contain 1 to 95% by volume of at least one type of component selected from inorganic substances and/or organic substances in addition to the biodegradable polyester resin.

4. A method for manufacturing a cosmetic product comprising microparticles and other cosmetic raw materials, said method comprising the steps of:
preparing a biodegradable polymer resin containing 50 mol% or more of lactic acid units, with a content of D-lactic acid among the lactic acid units being 0.1 to 25 mol%;
drying the biodegradable polyester resin to a moisture content of 1000 ppm or less;
applying a melt kneading method to the dried resin to form microparticles; and
adding the microparticles to the other cosmetic raw materials.
